# EUROPÄISCHE PATENTANMELDUNG

(11) **EP 1 188 452 A2**
(43) Veröffentlichungstag der Anmeldung: **20.03.2002**
(21) Anmeldenummer: 01121672.8
(22) Anmeldetag: 14.09.2001
(51) Int. Cl.: A61L 27/58, A61L 27/26, A61F 2/08

(54) **Medizintechnisches bioresorbierbares Implantat, Verfahren zur Herstellung und Verwendung**

(30) Priorität: 15.09.2000 DE 10046119
(71) Anmelder: DEUTSCHE INSTITUTE FÜR TEXTIL- UND FASERFORSCHUNG STUTTGART Stiftung des öffentlichen Rechts, 73770 Denkendorf (DE); Claes, Lutz, Prof.-Dr., 89233 Neu-Ulm/Pfuhl (DE)
(72) Erfinder: Dauner, Martin, Dr.-Ing., 73728 Esslingen (DE); Hierlemann, Helmut, Dr.-rer.nat., 73033 Göppingen (DE); Planck, Heinrich, Prof.Dr.-Ing., 72622 Nürtingen (DE); Claes, Lutz, Prof.Dr.human.biol., 89233 Neu-Ulm/Pfuhl (DE); Dürselen, Lutz, Dr.human.biol, 89073 Ulm (DE); Ignatius, Anita, Dr.-rer.nat., 89275 Elchingen (DE)
(74) Vertreter: Patentanwälte Ruff, Wilhelm, Beier, Dauster & Partner

(57) **Zusammenfassung**

Ein medizintechnisches bioresorbierbares Implantat, insbesondere für die Kreuzbandaugmentation ist ausgebildet als Verbundstruktur in textiler Konstruktion aus mindestens zwei bioverträglichen in ihrer chemischen Zusammensetzung und/oder Polymerstruktur verschiedenen Polymermaterialien, die degradierbar sind, wobei das Implantat eine vorbestimmte Anfangszugsteifigkeit besitzt und ein unterschiedliches Degradationsverhalten der Polymeren und/oder die textile Konstruktion so ausgewählt sind, dass die Zugsteifigkeit während der Degradation abnimmt.

## Beschreibung

Die vorliegende Erfindung betrifft ein medizintechnisches bioresorbierbares Implantat, ein Verfahrens zu seiner Herstellung und seine Verwendung in der Medizin.

Künstliche Implantate werden in der Chirurgie allgemein als Mittel zum Befestigen, Stützen oder als Ersatz für erkrankte oder verletzte Körperteile eingesetzt. Bekannt sind beispielsweise chirurgische Nähfäden, Ersatz für Sehen und Bänder sowie Gefässprothesen.

Speziell für Anwendungen in der Orthopädie von Mensch und Tier wurden künstliche Implantate in Form von Kordeln oder Bändern entwickelt. Ein typisches Beispiel für ihre Anwendung ist ein Kreuzbandriss, wo ein Implantat bei der Rekonstruktion oder als Prothese dienlich ist.

Da Implantate aus nichtresorbierbaren Materialien erhebliche Nachteile aufweisen, wie beispielsweise Fremdkörperreaktionen und Infektionsneigung, wurden Implantate aus teilweise oder vollständig resorbierbarem Material entwickelt. Aus DE-A1 19613730 der Firma Ethicon ist ein flächiges Implantat aus nicht oder langsam resorbierendem Material bekannt, das mit einem weiteren schnell resorbierenden Material versteift wird. Bekannt sind ferner verschiedene degradierbare Implantate, die auf Basis von Polyestern der alpha-Hydroxycarbonsäuren hergestellt sind. Die Firma Ethicon offenbart in DE-C2 4012602 Kordeln aus Polydioxanon.

Insbesondere bei Anwendungen, in denen das Implantat nur temporär seine Funktion ausüben soll, bis das körpereigene Gewebe sich erholt hat ist es wünschenswert, dass das eingesetzte Implantat nach und nach seine mechanische Stabilität verliert und damit zunehmend Last an das wiederherzustellende Gewebe übergibt.

In der Praxis weisen die bekannten Implantate den Nachteil auf, dass während der Degradationsphase nach einer anfänglichen Erweichung eine Versprödung erfolgt, die die Dehnungsfähigkeit des Implantats reduziert und entweder zur unerwünschten Dehnungsbeschränkung oder zum verfrühten Bruch des Implantats führt. Die funktionelle Belastung wird bei den bisherigen Implantaten erst dann vom körpereigenen wiederhergestellten Gewebe übernommen, wenn das Implantatmaterial so weit abgebaut ist, dass es zum Bruch des Implantats kommt.

Die Erfindung stellt sich daher die Aufgabe, ein medizintechnisches bioresorbierbares Implantat, insbesondere ein Augmentat zur Verfügung zu stellen, das gewünschte mechanische Eigenschaften zusammen mit einem vorteilhaften biologischen Degradations- und Resorptionsverhalten in vivo aufweist, das auch einfach und kostengünstig herzustellen ist sowie einfach und zuverlässig in der Anwendung bei Implantationen in der Chirurgie.

Diese Aufgabe wird gelöst durch ein medizintechnisches bioresorbierbares Implantat, insbesondere für die Kreuzbandaugmentation ausgebildet als Verbundstruktur in textiler Konstruktion aus mindestens zwei bioverträglichen in ihrer chemischen Zusammensetzung und/oder Polymerstruktur verschiedenen Polymermaterialien, die degradierbar sind, wobei das Implantat eine vorbestimmte Anfangszugsteifigkeit besitzt und ein unterschiedliches Degradationsverhalten der Polymeren und/oder die textile Konstruktion so ausgewählt ist, dass die Zugsteifigkeit während der Degradation abnimmt.

Das Implantat ist insbesondere als Augmentat für die Kreuzbandaugmentation ausgebildet. Durch die Abnahme der Zugsteifigkeit des Implantats wird erreicht, dass zunehmend Last auf das wiederherzustellende Gewebe übertragen wird.

Die Steifigkeit stellt in diesem Zusammenhang ein Mass für den Widerstand gegen Formänderungen dar. Sie kann als Steigung des Kraftverlaufs über der Verlagerung abgebildet werden. Ferner steht sie in Beziehung zum Elastizitätsmodul, der zusätzlich den Ausgangsquerschnitt und die Ausgangslänge des Probenmaterials berücksichtigt.

Bei der biologischen Resorption von natürlichen oder synthetischen Polymermaterialien in physiologischer Umgebung erfolgt zuerst eine Degradation der Polymerketten, wonach die Degradationsprodukte weiter abgebaut und schliesslich die Substanzmasse resorbiert wird. Eine Abnahme der Zugsteifigkeit der Polymere kann schon bei der Degradation auftreten, spätestens jedoch bei der Resorption der Abbauprodukte.

In einer besonderen Ausführungsform wird ein medizintechnisches bioresorbierbares Implantat zur Verfügung gestellt ausgebildet als Verbundstruktur in textiler Konstruktion aus mindestens drei oder mehr, bioverträglichen in ihrer chemischen Zusammensetzung und/oder Polymerstruktur verschiedenen Polymermaterialien, die degradierbar sind, wobei das Implantat eine vorbestimmte Anfangszugsteifigkeit besitzt und ein unterschiedliches Degradationsverhalten der Polymeren und/oder die textile Konstruktion so ausgewählt ist, dass die Zugsteifigkeit während der Degradation abnimmt. In der Regel reichen zwei oder drei verschiedene Polymermaterialien aus.

Mit besonderem Vorteil kann sich das erfindungsgemässe Implantat dadurch auszeichnen, dass sämtliche Polymere vollständig resorbierbar sind.

Gemäss der Erfindung kann die Abnahme der Zugsteifigkeit des Implantats durch die textile Konstruktion und die Degradierbarkeit von mindestens zwei der Polymeren bedingt sein.

Das Implantat kann eine Anfangszugsteifigkeit im Bereich von 50 bis 250 N/mm, insbesondere 80 bis 160 N/mm, bezogen auf eine Implantatlänge von 100 mm aufweisen. Die Steifigkeit des Implantatmaterials ist nicht auf den Querschnitt bezogen. Insbesondere kann die Steifigkeit mit abnehmendem Querschnitt geringer werden. Vorteilhaft kann sich das erfindungsgemässe Implantat dadurch auszeichnen, dass seine die Zugsteifigkeit bewirkenden Komponenten aus mindestens teilkristallinen Polymeren gebildet sind. Die Zugsteifigkeit des Implantats kann eine Halbwertszeit in vitro und in vivo von 3 bis 9 Monaten, insbesondere 5 bis 7 Monaten aufweisen.

In einer Ausführungsform des Implantats gemäss der Erfindung kann mindestens ein Polymer von hoher Steifigkeit eine schnellere Degradierbarkeit aufweisen als mindestens ein weiteres mit geringerer Steifigkeit. In einer besonderen Ausführungsform des Implantats gemäss der Erfindung können die verschiedenen Polymere eine etwa gleiche Steifigkeit, aber unterschiedliches Degradationsverhalten aufweisen.

Erfindungsgemäss kann ein langsam resorbierendes Polymer eine Resorptionszeit in vivo 6 bis 36 Monaten, insbesondere 12 bis 24 Monaten aufweisen. Ein schnell degradierendes Polymer kann eine Degradationszeit in vivo 3 bis 36 Wochen, insbesondere 12 bis 26 Wochen aufweisen. Ein auf diese Weise schnell degradierendes Polymermaterial verliert rasch seine Festigkeit, ist jedoch noch im Implantat vorhanden. Die bisher von einer schnell degradierenden Komponente übernommene Last, wird nun von anderen Komponenten des Implantats und/oder von natürlichem Körpergewebe übernommen. Auf diese Weise wird durch den teilweisen biologischen Abbau von Polymermaterial im Implantat sukzessive Platz geschaffen für nachwachsenden körpereigenes Gewebe, und zwar bevor die gesamte Festigkeit des Implantats verloren ist. Bisherige Implantate aus dem Stand der Technik hatten den Nachteil, dass im wesentlichen zuerst die Festigkeit verloren geht und dann Polymermassenverlust durch Resorption eintritt.

Die Halbwertszeiten der Degradation der verschiedenen Polymere im erfindungsgemässen Implantat können so aufeinander abgestimmt sein, dass, insbesondere nach einer Plateauphase, die Abnahme der Steifigkeit des Implantats über die Degradationszeit des Implantats möglichst stetig ist. Mit Vorteil kann die Halbwertszeit einer langsamer degradierenden Komponente um 25 bis 100 %, bevorzugt 50 bis 80 % länger sein als die der schneller degradierenden Komponente. Bei Verwendung von drei oder mehr Polymerkomponenten kann die Abstufung der Halbwertszeiten entsprechend angepasst sein. Das Degradationsverhalten der Polymeren kann durch ihre chemische Zusammensetzung und Polymerstruktur beeinflusst werden. Beispielsweise durch einen Gehalt an Monomer- oder Oligomerkomponenten. Ein anderer Einflussfaktor, durch dessen Variation das Abbauverhalten des erfindungsgemässen Polymers verändert werden kann, ist die Intensität und Dauer einer Gammabestrahlung wie sie beispielsweise zum Zwecke der Sterilisierung von medizintechnischem Material vorgenommen wird.

Erfindungsgemäss bevorzugt kann das langsamer degradierende Material ein grösseres oder gleiches Volumen im Implantat einnehmen, wie andere Materialien, um auf diese Weise eine ausreichende Festigkeit zu gewährleisten.

Die Verteilung der Degradation und Resorption der verschiedenen Polymere im erfindungsgemässen Implantat über einen längeren Zeitraum kann sich durch einen weiteren Vorteil auszeichnen, dass insbesondere bei Polyestern der alhpa-Hydroxycarbonsäuren, eine geringere Menge an Monomer pro Zeiteinheit an das Körpergewebe abgegeben wird, was eine Übersäuerung des umliegenden Gewebes vermeidet und damit für die Heilung förderlich ist.

Im Implantat gemäss der Erfindung kann die textile Konstruktion derart ausgebildet sein, dass durch die Degradation eines Materials die Struktur aus mindestens einem weiteren Material ein höheres Dehnungsvermögen erhält. Dies ist beispielsweise bei einem Mischgeflecht aus verschieden schnell degradierbaren Fäden der Fall.

In einer besonderen Ausführungsform des Implantats kann es in der textilen Konstruktion mindestens ein als mechanisch sperrendes Material dienendes Polymermaterial aufweisen, das schneller degradierbar ist als mindestens ein weiteres Polymer. Erfindungsgemäss kann das sperrende Material insbesondere ein Füllmaterial sein. im Implantat gemäss der Erfindung vorgesehenes Füllmaterial kann beispielsweise eine Seele sein. Mit Vorteil kann das Füllmaterial insbesondere als massives Material ausgebildet sein. In einer besonderen Ausführungsform der Erfindung braucht das Füllmaterial keine eigene Steifigkeit aufzuweisen. Als Füllmaterial kann beispielsweise ein Polymer mit weitgehend amorpher Struktur vorgesehen sein. Auch eine Imprägnierung eines Geflechts oder Gewirkes kann als anfängliche Dehnungssperre dienen.

In einer bevorzugten Ausführungsform der Erfindung kann das sperrende Material ein lastaufnehmendes, d. h. Zugspannung aufnehmendes, Material sein, das eine weniger steife Konstruktion und/oder anfänglich weniger steifes Polymermaterial entlastet. Mit Vorteil kann durch Entfernung, insbesondere Dagradation eines lastaufnehmenden Materials die textile Konstruktion höheres Dehnungsvermögen erhalten und/oder ein weniger steifes Polymermaterial die Last aufnehmen. Dies ist beispielsweise bei einem Geflecht der Fall, das axiale Stehfäden aus einem steifen Material aufweist, das relativ schnell degradierbar ist. Die anfänglich noch entlastete Geflechtsstruktur nimmt dann unter gleichzeitiger Dehnung zunehmend Last auf.

Das Dehnungsvermögen des erfindungsgemässen Implantats ist auf den vorgesehenen Anwendungsbereich abzustimmen. Die absolute Längenausdehnung des Implantats sollte nicht grösser sein als die natürliche Dehnbarkeit des zu unterstützenden Körperteils, z. B. der Sehne oder des Bandes. Vorzugsweise ist sie gleich der natürlichen Dehnbarkeit.

Das Implantat gemäss der Erfindung kann sich mit Vorteil dadurch auszeichnen, dass es aus Polymermaterial in Form von Filamenten gebildet ist. Insbesondere kann die textile Konstruktion mindestens teilweise von Filamenten gebildet sein. Die Filamente können sowohl als Einzelfilamente wie auch als Multifilamente ausgebildet sein. In einer Ausführungsform der Erfindung können die Filamente jeweils aus nur einem Polymermaterial gebildet sein. In einer bevorzugten Ausführungform können die Filamente als Mehrkomponentenfasern, insbesondere als Bikomponentenfasern ausgebildet sein. Ausserdem können Multifilamente aus einer Mischung von Filamenten unterschiedlicher Polymermaterialien ausgebildet sein. Vorteilhaft können Filamente im Implantat einen Durchmesser von 6 bis 30 um, insbesondere 10 bis 17 µm aufweisen. Polymerfilamente können nach bekannten Techniken verstreckt und thermisch nachbehandelt sein, um optimale Fasereigenschaften für die Anwendung zu erzielen. Wie den Fachleuten bekannt ist, fördert ein Verstreckvorgang die Orientierung der Polymermoleküle im Filament und die Ausbildung kristalliner Strukturmerkmale. Aufgrund der Korrelation von Struktur und Eigenschaften bei Polymeren kann über den Verstreckungsgrad die Zugsteifigkeit von Polymerfasern beeinflusst werden.

Bevorzugt kann das erfindungsgemässe Implantat so ausgebildet sein, dass mindestens ein Teil der Filamente texturiert ist. Eine Texturierung von Filamenten im erfindungsgemässen Implantat kann nach üblichen Techniken vorgenommen sein. Insbesondere können die Filamente gefacht sein. In einer Ausführungsform der Erfindung können die schneller degradierenden Filamente texturiert sein. In einer bevorzugten Ausführungsform können die langsamer degradierenden Filamente texturiert sein. Texturierte Fasern im Implantat können die Dehnfähigkeit günstig beeinflussen. Ausserdem können texturierte Fasern ein Einwachsen von natürlichem Körpergewebe begünstigen.

Gemäss einer Ausführungsform der Erfindung kann im Implantat die Verbundstruktur linienförmig ausgebildet sein, insbesondere als Strang, Schnur, Kordel, Band oder Seil. Eine solche Ausführungsform kann mit besonderem Vorteil für eine chirurgische Behandlung an Sehnen und Bändern eines menschlichen oder tierischen Patienten verwendet werden.

In einer besonderen Ausführungsform der Erfindung kann im Implantat die Verbundstruktur als Flächengebilde ausgebildet sein. Eine solche flächige Ausführungsform ist beispielsweise insbesondere für eine Anwendung als Herniennetz vorteilhaft.

In einer bevorzugten Ausführungsform der Erfindung kann im Implantat die Verbundstruktur verzweigt ausgebildet sein. Eine solche Ausführungsform ist beispielsweise besonders für eine Anwendung beim vorderen Kreuzband vorteilhaft.

In einer weiteren besonderen Ausführungsform der Erfindung kann im Implantat die Verbundstruktur als dreidimensionaler Körper ausgebildet sein. Eine solche Ausführungsform ist beispielsweise besonders für eine Anwendung bei der Osteosynthese vorteilhaft.

Insbesondere kann eine Verbundstruktur einlagig ausgebildet sein. Ein Beispiel für eine solche Verbundstruktur ist ein Geflecht aus einer Garnmischung.

Bevorzugt kann eine Verbundstruktur mehrlagig ausgebildet sein. Als ein Beispiel für eine mehrlagige Verbundstruktur gemäss der Erfindung sind zwei ineinander angeordnete Schläuche zu nennen.

Erfindungsgemäss kann im Implantat die Verbundstruktur in einer einzigen textilen Technik ausgebildet sein. Bevorzugt kann die Verbundstruktur in einer Kombination von textilen Techniken ausgebildet sein.

In einer Ausführungsform kann die Verbundstruktur mindestens teilweise als gewebte Konstruktion, insbesondere mit Kettfäden in Längsrichtung ausgebildet sein.

In einer besonderen Ausführungsform kann die Verbundstruktur mindestens teilweise als Maschenware, insbesondere als gewirkte Konstruktion ausgebildet sein.

In einer bevorzugten Ausführungsform kann die Verbundstruktur mindestens teilweise als geflochtene Konstruktion, insbesondere als Spiralgeflecht ausgebildet sein. In einer besonders bevorzugten Ausführungsform kann das Spiralgeflecht mit Stehfäden aus elastomerem Material ausgebildet sein. In Weiterbildung kann die Verbundstruktur mindestens teilweise als dreidimensionales Geflecht ausgebildet sein. In einer anderen besonderen Ausführungsform kann ein Geflecht aus Bikomponentenfasern ausgebildet sein, wobei insbesondere die schneller degradierende Komponenten die langsamer degradierende Komponente umhüllt.

In einer besonders bevorzugten Ausführungsform kann das Implantat mindestens teilweise als Verbundstruktur vom Typ Seele-Mantel ausgebildet sein, vorzugsweise mit mindestens zwei Mänteln.

Mit Vorteil kann die textile Gestaltung des Implantats derart sein, dass Polymerfasern nicht vornehmlich in Richtung der Längsachse des Implantats, sondern in einem Winkel im Bereich zwischen 5° bis 60° (Winkelgrad) zur Längsachse angeordnet sind. Dies ermöglicht eine Strukturdehnung und bei Degradierung und Resorption einer Polymerkomponente kann eine Steifigkeitsabnahme des gesamten Systems erreicht werden. Auf diese Weise kann während der Degradationsphase die Steifigkeit des Implantats sukzessive abnehmen, ebenso die Festigkeit, wobei die Reissdehnung noch in etwa erhalten bleibt. Für einen optimalen Behandlungserfolg sollte die Reststeifigkeit hoch genug sein, dass keine Überdehnung natürlicher Körpergewebe, wie eines Bandes oder einer Sehne auftreten kann. Das Implantat kann so bis in die späte Resorptionsphase das sich regenerierende Körpergewebe unterstützen, ohne es zu sehr zu entlasten, und damit die volle Funktionsfähigkeit des regenerierten Gewebes fördern. In der Heilungsphase kann das Implantat unphysiologisch grosse Verformungen auffangen.

Besonders für die Behandlung von Kreuzbändern ist ein als Geflecht ausgebildetes Implantat in entsprechend ausgebildeter Verbundstruktur bevorzugt. Ein als Geflechtkonstruktion ausgebildetes Implantat weist eine für diesen Anwendungsfall vorteilhafte Strukturdehnung auf.

Gegenstand der vorliegenden Erfindung ist ferner ein Verfahren zur Herstellung eines medizintechnischen bioresorbierbaren Implantats, insbesondere für die Kreuzbandaugmentation dadurch gekennzeichnet, dass zwei oder mehrere bioverträgliche in ihrer chemischen Zusammensetzung und/oder ihrer Polymerstruktur verschiedene Polymermaterialien, die degradierbar sind, und sich mindestens zwei der Verbundkomponenten in ihrem Degradationsverhalten unterscheiden, nach textilen Verarbeitungstechniken mindestens eines Polymermaterials derart zu einer Verbundstruktur geformt werden, dass es eine vorbestimmte Anfangszugsteifigkeit erhält und die Steifigkeit des Implantats während der Degradation abnimmt.

Ausserdem ist Gegenstand der vorliegenden Erfindung die Verwendung des erfindungsgemässen medizintechnischen Implantats als Implantat für die Chirurgie, insbesondere zur Augmentation von Kreuzbändern.

Für die Anwendung als Augmentationskordel ist es vorteilhaft, wenn über die Zeit die Steifigkeit des künstlichen Implantatmaterials gegenüber dem natürlichen körpereigenen Material abnimmt. Abnehmende Steifigkeit kann am Anfang elastisch erfolgen oder auch durch einfache (zugfreie) Verlängerbarkeit. Beispielsweise bei einem Geflecht aus Mischfasern nach Degradation und Resorption eines Fasermaterials.

Für die Anwendung als Herniennetz wird durch dieses ein Narbengewebe erzeugt, das die Funktion der Fasciae übernimmt. Auch hier ist eine degradierende Steifigkeit erwünscht.

Für eine optimale Anwendung kann ein erfindungsgemässes Implantat mit Strukturen kombiniert sein, die beispielsweise der Verankerung im Körper des Patienten dienen, wie etwa Ösen. Ferner können Hilfsstoffe und/oder medizinische Wirkstoffe mit dem Implantat kombiniert sein, um den Behandlungserfolg zu fördern.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

### Beispiel 1

### Vergleich der Eigenschaften bioresorbierbarer Polymere

Die folgende Tabelle gibt Daten zu den physikalischen und mechanischen Eigenschaften sowie dem Degradations- und Resorptionsverhalten verschiedener biokompatibler Polymere auf Basis von Glykolid, Lactid sowie Hydroxycarbonsäure an.

| Polymer | Schmelz bereich [°C] | Glaspunkt [°C] | Prozesstemperatur [°C] | Faser-Zugfestigkeit [MPa] | Degradation Wochen | Resorption Wochen |
|---|---|---|---|---|---|---|
| Polyglykolsäure (PGA) | 215 - 226 | 36 | 230 - 260 | 760 - 920 | 4 - 5 | 7 - 20 |
| Poly-L-Lactid (P-L-LA) Poly-D-Lactid (P-D-LA) | 175 - 180 | 55 - 60 | 180 - 230 | 600 - 800 | 16 - 52 | 40 - > 104 |
| Poly-DL-Lactid (P-DL-LA) | amorph | 50 | 150 - 190 | 250 - 350 | 15 - 30 | 25 - 52 |
| Poly-L/DL-Lactid 70:30 P-L/DL-LA 70:30 | amorph | 52 | 170 - 200 | 300 - 450 | 18 - 36 | 36 - 60 |
| Poly-(glykolid-co-L-Lactid) 90/10 VICRYL® | 198 | 36 - 45 | 200 - 250 | 570 - 910 | 4 - 5 | 13 - 18 |
| Poly-(L-Lactid-co-glykolid) ("LGA 90/10") | 156 | 54 | 180 - 220 | 500 - 750 | 10 - 20 | 30 - 52 |
| Poly-β-hydroxycarbonsäure | 175 | 5 | > 190 | n.a. | 25 - 75 | 50 - 100 |

### Beispiel 2

### Herstellung eines zweilagigen Geflechts aus Fasermischungen

Garne aus den Copolymeren Poly-L-Lactid-co-glycolid im Monomerverhältnis 10/90 (LGA 1090), Poly-L-Lactid-co-glycolid im Monomerverhältnis 90/10 (LGA 9010) und Poly-L-Lactid (P-L-LA) mit einer Feinheit von jeweils etwa 330 dtex werden gefacht. Sie werden mit acht Klöppeln zu einem Kerngeflecht verarbeitet, welches mit einem Mantel aus der gleichen Garnmischung mit 16 Klöppeln umhüllt wird. Die Anfangsfestigkeit beträgt 655 N bei 17 % Reissdehnung. Die Steifigkeit nimmt von 116 N/mm über einen Zeitraum von 12 Monaten degressiv gegen den Wert 0 ab.

### Beispiel 3

### Herstellung eines zweilagigen Geflechts aus unterschiedlichen Polymermaterialien

Ein Kerngeflecht auf schneller degradierendem LGA 1090 (8 Kl x 800 dtex) wird mit einem 16 Klöppel-Geflecht aus langsamer degradierendem LGA 9010 und P-L-LA, jeweils 840 dtex, umflochten, wobei jeweils 8 Klöppel mit einem Material bestückt sind. Die Anfangsfestigkeit beträgt 529 N bei 17 % Dehnung. Der Verlauf der Steifigkeit über der Degradationszeit ist qualitativ dem von Beispiel 1 vergleichbar, jedoch beträgt die Anfangssteifigkeit 95 N/mm, der Gradient ist geringer.

### Beispiel 4

### Herstellung eines zweilagigen Geflechts aus unterschiedlichen Polymermaterialien

Ein Kerngeflecht aus langsamer degradierendem PLLA (8 Kl x 800 dtex) wird mit einem 16 Klöppel-Geflecht aus etwas schneller degradierendem LGA 9010 (840 dtex) umflochten. Die Anfangsfestigkeit beträgt 632 N bei 18 % Dehnung. Der Verlauf der Steifigkeit über der Degradationszeit ist stufenweise. Die Reissdehnung bleibt lange konstant, jedoch nimmt die Festigkeit ab. Diese Konstruktion hat den Vorteil, dass die schneller degradierende Komponente aussen ist und daher Degradationsprodukte bei der Degradation schneller von der Körperflüssigkeit absorbiert werden.

### Beispiel 5

### Dreifach-Geflecht

Ein dreifach Gefelcht wird von innen nach aussen gebildet aus: LGA 1090 (8 Kl x 960 dtex), LGA 9010 (12 Kl x 960 dtex) und P-L-LA (24 Kl x 960 dtex). Pro Geflechtlage wird nur ein Garnmaterial verwendet. Die Degradationsgeschwindigkeit nimmt von innen nach aussen ab. Die Anfangsfestigkeit beträgt 551 N bei 12 % Dehnung, die Steifigkeit nimmt von 145 N/mm fast linear ab.

### Beispiel 6

### Dreifache Seele-Mantel-Konstruktion

Eine dreifache Konstruktion wird aus einer monofilen Seele aus einem degradierbaren Glykolsäure/Trimethylencarbonat-Copolymer 55/45 umflochten mit LG 9010 (12 Kl x 960 dtex) und als äusserer Mantel P-L-LA (24 Kl x 960 dtex) ausgebildet. Bei einer Anfangsfestigkeit von 481 N und 13 % Dehnung nimmt hier die Steifigkeit von 123 N/mm progressiv über 12 Monate ab.

## Patentansprüche

1. Medizintechnisches bioresorbierbares Implantat, insbesondere für die Kreuzbandaugmentation ausgebildet als Verbundstruktur in textiler Konstruktion aus mindestens zwei bioverträglichen in ihrer chemischen Zusammensetzung und/oder Polymerstruktur verschiedenen Polymermaterialien, die degradierbar sind, wobei das Implantat eine vorbestimmte Anfangszugsteifigkeit besitzt und ein unterschiedliches Degradationsverhalten der Polymeren und/oder die textile Konstruktion so ausgewählt sind, dass die Zugsteifigkeit während der Degradation abnimmt.

2. Implantat nach Anspruch 1, **dadurch gekennzeichnet, dass** die Abnahme der Zugsteifigkeit durch die textile Konstruktion und die Degradierbarkeit von mindestens zwei der Polymeren bedingt ist.

3. Implantat nach Anspruch 1 oder 2, **dadurch gekennzeichnet, dass** es eine Anfangszugsteifigkeit im Bereich von 50 bis 250 N/mm, insbesondere 80 bis 160 N/mm, bezogen auf eine Implantatlänge von 100 mm aufweist.

4. Implantat nach einem der Ansprüche 1 bis 3, **dadurch gekennzeichnet, dass** seine Zugsteifigkeit eine Halbwertszeit in vitro und in vivo von 3 bis 9 Monaten, insbesondere 5 bis 7 Monaten aufweist.

5. Implantat nach einem der vorhergehenden Ansprüche, **dadurch gekennzeichnet, dass** mindestens ein Polymer von hoher Steifigkeit eine schnellere Degradierbarkeit aufweist als mindestens ein weiteres mit geringerer Steifigkeit.

6. Implantat nach einem der Ansprüche 1 bis 4, **dadurch gekennzeichnet, dass** die verschiedenen Polymere eine etwa gleiche Steifigkeit, aber unterschiedliches Degradationsverhalten aufweisen.

7. Implantat nach einem der vorhergehenden Ansprüche, **dadurch gekennzeichnet, dass** die textile Konstruktion derart ausgebildet ist, dass durch die Degradation eines Materials die Struktur aus mindestens einem weiteren Material ein höheres Dehnungsvermögen erhält.

8. Implantat nach einem der vorhergehenden Ansprüche, **dadurch gekennzeichnet, dass** es in der textilen Konstruktion mindestens ein als mechanisch sperrendes Material dienendes Polymermaterial aufweist, das schneller degradierbar ist als mindestens ein weiteres Polymer.

9. Implantat nach einem der vorhergehenden Ansprüche, **dadurch gekennzeichnet, dass** das sperrende Material ein Füllmaterial ist.

10. Implantat nach einem der vorhergehenden Ansprüche, **dadurch gekennzeichnet, dass** das sperrende Material ein lastaufnehmendes Material ist, das eine weniger steife Konstruktion und/oder anfänglich weniger steifes Polymermaterial entlastet.

11. Implantat nach einem der vorhergehenden Ansprüche, **dadurch gekennzeichnet, dass** durch Entfernung, insbesondere Degradation eines lastaufnehmenden Materials die textile Konstruktion höheres Dehnungsvermögen erhält und/oder ein weniger steifes Polymermaterial die Last aufnimmt.

12. Implantat nach einem der vorhergehenden Ansprüche, **dadurch gekennzeichnet, dass** Filamente jeweils aus nur einem Polymermaterial gebildet sind.

13. Implantat nach einem der Ansprüche 1 bis 11, **dadurch gekennzeichnet, dass** Filamente als Mehrkomponentenfasern, insbesondere als Bikomponentenfasern ausgebildet sind.

14. Implantat nach einem der vorhergehenden Ansprüche, **dadurch gekennzeichnet, dass** Multifilamente aus einer Mischung von Filamenten unterschiedlicher Polymermaterialien gebildet sind.

15. Implantat nach einem der vorhergehenden Ansprüche, **dadurch gekennzeichnet, dass** die Verbundstruktur linienförmig ausgebildet ist, insbesondere als Strang, Schnur, Kordel, Band oder Seil.

16. Implantat nach einem der vorhergehenden Ansprüche, **dadurch gekennzeichnet, dass** die Verbundstruktur mehrlagig ausgebildet ist.

17. Implantat nach einem der vorhergehenden Ansprüche, **dadurch gekennzeichnet, dass** die Verbundstruktur in einer Kombination von textilen Techniken ausgebildet ist.

18. Implantat nach einem der vorhergehenden Ansprüche, **dadurch gekennzeichnet, dass** die Verbundstruktur mindestens teilweise als geflochtene Konstruktion, insbesondere als Spiralgeflecht ausgebildet ist.

19. Implantat nach einem der vorhergehenden Ansprüche, **dadurch gekennzeichnet, dass** die Verbundstruktur mindestens teilweise als dreidimensionales Geflecht ausgebildet ist.

20. Implantat nach einem der vorhergehenden Ansprüche, **dadurch gekennzeichnet, dass** es mindestens teilweise als Verbundstruktur vom Typ Seele-Mantel ausgebildet ist, vorzugsweise mit mindestens zwei Mänteln.

21. Verfahren zur Herstellung eines medizintechnischen bioresorbierbaren Implantats, insbesondere für die Kreuzbandaugmentation **dadurch gekennzeichnet, dass** zwei oder mehrere bioverträgliche in ihrer chemischen Zusammensetzung und/oder ihrer Polymerstruktur verschiedene Polymermaterialien, die degradierbar sind, und sich mindestens zwei der Verbundkomponenten in ihrem Degradationsverhalten unterscheiden, nach textilen Verarbeitungstechniken mindestens eines Polymermaterials derart zu einer Verbundstruktur geformt werden, dass es eine vorbestimmte Anfangszugsteifigkeit erhält und die Steifigkeit des Implantats während der Degradation abnimmt.

22. Bereitstellung des medizintechnischen Implantats nach einem der vorhergehenden Ansprüche als Implantat für die Chirurgie, insbesondere zur Augmentation von Kreuzbändern.
